(19) Europäisches Patentamt
European Patent Office
Office européen des brevets

(11) **EP 3 637 431 B1**

(12) **EUROPEAN PATENT SPECIFICATION**

(45) Date of publication and mention
of the grant of the patent:
**17.09.2025 Bulletin 2025/38**

(21) Application number: **18200109.9**

(22) Date of filing: **12.10.2018**

(51) International Patent Classification (IPC):
***G16H 50/20*** *(2018.01)*

(52) Cooperative Patent Classification (CPC):
**G16H 50/20**

(54) **MEDICAL DIAGNOSTIC AID AND METHOD**

MEDIZINISCHE DIAGNOSEHILFE UND VERFAHREN

AIDE AU DIAGNOSTIC MÉDICAL ET PROCÉDÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**15.04.2020 Bulletin 2020/16**

(73) Proprietor: **FUJITSU LIMITED
Kanagawa 211-8588 (JP)**

(72) Inventors:
• **GARCIA SANTA, Nuria
28010 Madrid (ES)**

• **VILLAZÓN-TERRAZAS, Boris
28003 Madrid (ES)**

(74) Representative: **Haseltine Lake Kempner LLP
Cheapside House
138 Cheapside
London EC2V 6BJ (GB)**

(56) References cited:
**EP-A1- 3 223 177     US-A1- 2015 095 016
US-A1- 2015 379 241**

**Description**

. The invention relates to a medical diagnostic aid and method for assisting medical personnel in performing a diagnosis. Also disclosed is a computer program code which, when executed by a computer, causes the computer to perform the method, and a non-transitory computer readable medium comprising the computer program.

. Natural Language Processing (NLP) is a way for computers to analyse, understand, and derive meaning from natural human language (as opposed to machine language, such as computer code). NLP is a very important field of research focused in the combination of formal theories, statistical data, machine learning and highlighting the use of semantics and contextualisation to extract the meaning of the texts.

. One area of human activity to which NLP is particularly well suited is medical practice and research. The clinical and research medical community creates, manages and uses a wide variety of semi-structured and unstructured textual documents. As such, NLP and Text Mining have become crucial tools in healthcare and the life sciences. The patients' medication histories and their responses during, for example, consultations with medical practitioners may be used to inform future medical treatment. However, currently this information is typically recorded in non-standardised forms, which may increase the difficulty in subsequently retrieving useful information from records of past consultations.

. As more and more information is available in the Electronic Health Records (EHRs) in the form of free-text narrative, there is a need for automated tools, which may process and understand such texts. Currently doctors and medical professionals spend a lot of time on processing free text medical notes. Due to time pressures, it is common for medical professionals not to be able to dedicate sufficient time to processing notes. Another important input in healthcare is the doctor-patient conversation as diagnostic assessment, which is key to evaluate the issues and needs of a patient. Again, it is common for recording of the interaction between doctor and patient to be incompletely documented, which may negatively impact upon the treatment received by patients.

. It is desirable to provide an automated system capable of processing large volumes of unstructured and semi-structured clinical content and deriving concepts, structure, and relationships from it. Automatic mapping of key concepts from clinical notes to a terminology is an important task to achieve for extraction of the clinical information locked in clinical notes and patient reports. There are potential benefits from such a system both on an individual patient level, and on a general practice level. US 2015/0095016 A1 discloses computer implemented systems and methods of processing clinical documentation for multi-axial coding schemes. The methods involve inputting clinical documentation into a computer system from a linked memory, and processing narrative text in the clinical documentation using a natural language processor. The processor is configured to segment the narrative text based on boundaries defined in the clinical documentation, sequences words in the narrative text based on the segmentation, and maps the sequenced words to semantic objects in an ontology database. The ontology defines classes of semantic objects and relationships between them, corresponding to the multi-axial coding scheme. The sematic objects are converted into characters and output into slots in a medical code, with the characters positioned in the slots based on the multi-axial coding scheme.

. An aspect of an embodiment provides a medical diagnostic apparatus for assisting medical personnel in performing a diagnosis, the diagnostic apparatus comprising: a receiver configured to receive an unstructured input data or information (hereinafter, more simply referred to as "unstructured input"); an analyser and parser configured to receive the unstructured input from the receiver, to split the unstructured input into a plurality of logical components, wherein the logical components are term groupings, and to detect medical terms in the plurality of logical components using a neural network; a synonym mapping engine configured to receive a medical classification hierarchy of medical standard codes in the form of a knowledge graph, and semantically annotate the knowledge graph with synonyms of medical terms used in the medical standard codes to create an enhanced knowledge graph; an automatic coding solver configured: to receive the enhanced knowledge graph from the mapping engine; to compare the medical terms detected in the plurality of logical components by the analyser and parser against the enhanced knowledge graph using a string matching algorithm; to generate a list of potential matching medical standard codes for each of the medical terms based on the comparison with the enhanced knowledge graph; to compare the lists of potential matching medical standard codes with each other; and to output top matching medical standard codes based on the most commonly occurring potential matching medical standard codes identified in the comparison of the potential matching medical standard codes; and an enrichment engine comprising a database of diagnoses linked to symptoms and treatments, wherein the enrichment engine is configured to compare the top matching medical standard codes output by the automatic coding solver against entries in the database of diagnoses, and to output diagnoses, symptoms and treatments related to each of the top matching medical standards codes for assisting medical personnel in providing a diagnosis. By automating the analysis of the unstructured input and providing diagnosis, symptom and treatment information for review by a medical practitioner, the medical diagnostic apparatus may save time and effort for the medical practitioner, and also provide the medical practitioner with information which may not otherwise have been discovered by the medical practitioner.

. The automatic coding solver may be further configured to apply a matching threshold that is a threshold for the similarity between two potential matching medical standard codes when comparing the lists of potential matching medical standard codes, thereby ensuring the relevance of the top matching medical standard codes output by the automatic coding solver. Further, if none of the comparisons of potential matching medical standard codes satisfies the threshold, a null result may be output to the enrichment engine as the top matching medical standard code comparison result, thereby reducing the chances of incorrect top matching medical standard codes being output to the enrichment engine and improving the reliability of the generated results. Alternatively, if none of the comparisons of potential matching medical standard codes satisfies the threshold, the highest ranking comparison below the threshold may be output to the enrichment engine as the top matching medical standard code comparison result, thereby reducing the chances of information being lost.

. The enrichment engine may be further configured to access a specific medical record linked to a patient that is the subject of the unstructured input, and to output the specific medical record in conjunction with the output diagnoses, symptoms and treatments, thereby providing additional useful information for review by medical personnel. Further, the enrichment engine may be configured to analyse the specific medical record, to extract medical standard codes from the specific medical record, and to take the extracted medical standard codes into account when outputting the diagnoses, symptoms and treatments. In this way, historical information relating to the patient may be taken into account and more reliable diagnostic apparatus may be provided to the medical personnel. Also, the enrichment engine may be configured to update the specific medical record with the output diagnoses, symptoms and treatments, again saving the labour of medical personnel.

. The enrichment engine may be further configured to output the unstructured input for evaluation by medical personnel, thereby allowing easy verification of the output from the diagnostic apparatus. The output diagnoses, symptoms and treatments and unstructured input may be stored in a storage device, for use in a training data set.

. The medical diagnostic apparatus may be further configured such that: either the automatic coding solver is configured to output top matching codes separately for each of the logical components based on the most commonly occurring potential matching medical standard codes identified for each of the logical components, and the enrichment engine is configured to compare the top matching medical standard codes output by the automatic coding solver against entries in the database of diagnoses separately for each of the logical components, and to output diagnoses, symptoms and treatments linked to each of the top matching medical standards codes separately for each of the logical components, or the automatic coding solver is configured to output consolidated top matching codes for the unstructured input as a whole based on the most commonly occurring potential matching medical standard codes identified for the unstructured input, and the enrichment engine is configured to compare the top matching medical standard codes output by the automatic coding solver against entries in the database of diagnoses, and to output diagnoses, symptoms and treatments linked to the top matching medical standards codes, for the unstructured input as a whole. Outputting at a logical component level may increase the chances of detection of plural medical conditions if present, and outputting consolidated results per unstructured input may increase the accuracy of the outputted results.

. The automatic coding solver may be configured: when analysing the potential matching medical standard codes, to divide the potential matching medical standard codes into phrase categories: first phrases used in the description of a medical standard code; second phrases that are synonyms of terms used in the description of a medical standard code; and third phrases that are healthcare related phrases, but are neither first phrases or second phrases, and when generating a list of top matching medical standard codes, the first phrases may be given greater weight than the second phrases, and the second phrases may be given greater weight than the third phrases. In this way, information which is likely to be of more use for generating diagnosis information is passed to the enrichment engine.

. The unstructured input received by the receiver may be an audio file containing a recording of a patient consultation, and the receiver may comprise a transcription module configured to generate a text file based on the audio file. By configuring the receiver to be able to operate with inputs of different types, the versatility of the system is improved.

. An aspect of an embodiment provides a method for assisting medical personnel in performing a diagnosis, the method comprising: receiving an unstructured input; splitting the received unstructured input into a plurality of logical components, wherein the logical components are term groupings, and detecting medical terms in the plurality of logical components using a neural network; receiving a medical classification hierarchy of medical standard codes in the form of a knowledge graph, and semantically annotating the knowledge graph with synonyms of medical terms used in the medical standard codes to create an enhanced knowledge graph; analysing the medical terms detected in the plurality of logical components against the enhanced knowledge graph using a string matching algorithm, generating a list of potential matching medical standard codes for each of the medical terms based on the comparison with the enhanced knowledge graph, comparing the lists of potential matching medical standard codes with each other, and outputting top matching medical standard codes based on the most commonly occurring potential matching medical standard codes identified in the comparison of the potential matching medical standard codes; comparing the top matching medical standard codes output against entries in a database of diagnoses related to symptoms and treatments, and outputting diagnoses, symptoms and treatments linked to each of the top matching medical standards codes for assisting medical personnel in providing a diagnosis. As with the diagnostic apparatus, the method may save time and effort for the medical practitioner, and also provide the medical practitioner with information which may not otherwise have been discovered by the medical practitioner.

. An aspect of an embodiment provides a non-transitory computer readable medium comprising code which, when executed by a computer, causes the computer to execute the method. This provides a convenient way to implement the method.

**Description of Figures**

. The invention is described, by way of example only, with reference to the following Figures, in which:

> Fig. 1 is a schematic diagram of an apparatus in accordance with an embodiment.
> Figs. 2A and 2B are a flowchart of a method in accordance with an embodiment.
> Fig. 3 is a diagram of a biomedical entity matrix.
> Fig. 4 is a block diagram of a computing device which embodies an embodiment.

**Detailed Description**

. Aspects of embodiments utilise Medical Classification (also referred to as Medical Coding), which is the process of transforming descriptions of medical diagnoses and procedures into standardised and universal medical code numbers. The diagnoses and procedures are usually taken from a variety of sources within the health care record, such as the transcription of the physician's notes and medical conversations between doctors and patients, laboratory results, radiologic results, and so on. Diagnosis codes track diseases and other health conditions. Procedure codes track interventions performed. These diagnosis and procedure codes are used by health care providers, government health programs, private health insurance companies, workers' compensation carriers and others for a variety of applications in medicine, public health and medical informatics.

. Any suitable Medical Classification scheme may be used; an example of a suitable scheme is the World Health Organisation (WHO) International Statistical Classification of Diseases and Related Health Problems (ICD) scheme, for which the 10th revision (ICD-10) is currently in active use (see http://apps.who.int/classifications/icd10/browse/2016/en for an online version of ICD-10, active as of 9 October 2018). Various derived schemes, such as the International Classification of Diseases for Oncology, third edition (ICD-O-3) may also be utilised (see http://codes.iarc.fr/usingicdo.php for an online version of ICD-O-3, active as of 9 October 2018). The ICD is a health care classification system that provides a system of diagnostic codes for classifying diseases, including nuanced classifications of a wide variety of signs, symptoms, abnormal findings, complaints, social circumstances, and external causes of injury or disease.

. Aspects of embodiments are configured to receive unstructured inputs comprising medical information relating to at least one patient. The unstructured inputs are then processed and used to generate medical standard code numbers. The generated code numbers may then be compared to a database of diagnoses, and used to generate potential diagnoses, symptoms and treatments linked to the generated code numbers. The diagnoses, symptoms and treatments may then be output and reviewed by suitably trained medical personnel, and thereby provide assistance in diagnosing a patient. In addition to providing assistance in diagnosing a patient, the output information may also be used to identify symptoms which may be tested for in order to confirm or refute potential diagnoses, and also may provide suggestions of treatment options which may be utilised.

. To be effective, it is necessary for the system to be able to automatically identify and match medical terms and entities with codes for different medical classifications, in order to speed up diagnoses and save time of physicians, managers and health professionals in general. In order to fully satisfy this role, a number of different components are required.

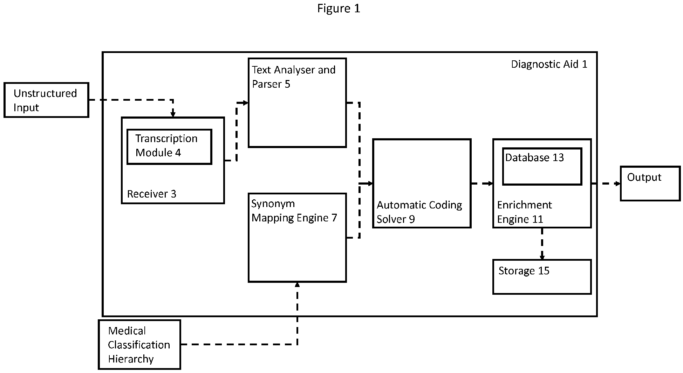

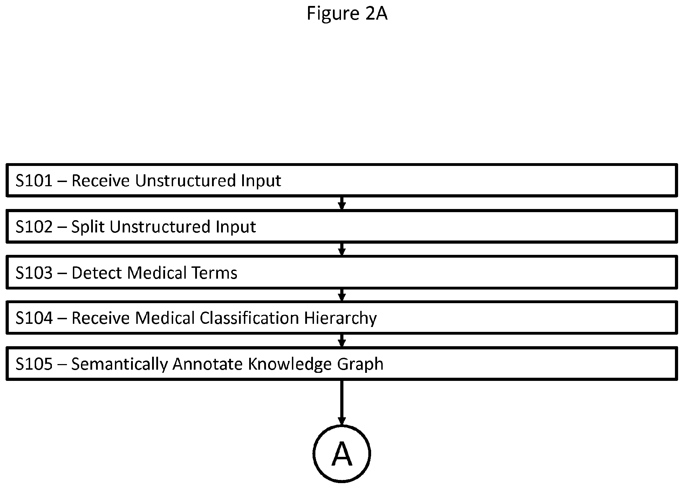

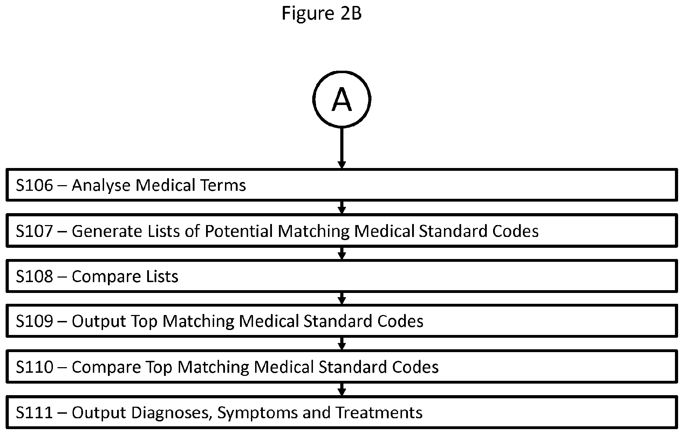

. Fig. 1 shows an overview of the components in a diagnostic aid 1 in accordance with an aspect of an embodiment. The diagnostic aid 1 shown in Fig. 1 comprises: a receiver 3 (including a transcription module 4), an analyser and parser 5, a synonym mapping engine 7, an automatic coding solver 9, an enrichment engine 11 (including a database 13) and storage 15. The components are discussed in detail below. Further, Figs. 2A and 2B show a flowchart detailing a method in accordance with an aspect of an embodiment.

. The diagnostic aid 1 comprises a receiver 3 that is configured to receive an unstructured input, as shown in step S101 of Fig. 2A. The unstructured input comprises medical information relating to at least one patient. Although information relating to a plurality of patients may be combined into a single unstructured input, typically separate inputs are used for each patient for simplicity when associating standard medical codes with patients as discussed below. The inputs are referred to as "unstructured" because no specific template is followed.

. The receiver 3 may be configured to receive the unstructured input in a variety of different forms, depending on the type of unstructured input provided. Although the system may be configured to receive inputs from, for example, diagnostic tests, ECGs, etc., typically the unstructured inputs are records of patient consultations or clinical notes. The unstructured inputs generated from patient consultations or clinical notes are usually provided as either written information or as an audio recording. Audio recordings may comprise direct recordings of conversations between a medical practitioner and a patient, comments dictated by a medical practitioner (often following a consultation), or a combination of the two. Similarly, clinical notes may be either recorded during examinations (that is, clinical rounds), subsequently to the rounds, or a combination of the two. Where unstructured inputs are provided in written form, the inputs may be hand written or typed.

. The diagnostic aid 1 is configured to operate using written information. As such, when unstructured inputs are input into the receiver 3, the receiver 3 may be configured to convert the unstructured inputs into a suitable format if necessary. For example, in aspects of embodiments configured to operate with unstructured inputs that are provided as audio files, the receiver 3 may comprise a transcription module 4 that is configured to convert audio files into text files. Further, in aspects of embodiments wherein handwritten unstructured inputs are provided (for example, as scans of handwritten pages), the receiver 3 may be further configured to perform text recognition to generate a text file.

. The unstructured input received by the receiver 3 is then passed on to an analyser and parser 5, typically configured to operate using text unstructured inputs. The text analyser and parser 5 is configured to receive an unstructured input text file from the receiver 3 (the text file may have been generated from a further file type as discussed above). The text analyser is then configured to split the unstructured input into a plurality of logical components, as shown in step S102 of Fig. 2A. Typically the logical components are sentences, although other logical components may also be used. For example, where the text input file contains very long or very short sentences, or no punctuation, it may be appropriate to divide the text file into term groupings in another way.

. Once the text input has been divided into logical components, the text analyser and parser 5 is configured to detect and extract from each logical component of the text any medical terms and entities that may be present (see step S103 of Fig. 2A). For this task, the text analyser and parser 5 is configured to use technologies such as OpenNLP frameworks, as are familiar to those working in the technical field. Part-of-Speech (POS) solutions may also be used to identify the type of the words; of particular relevance are terms of type NN (i.e. substantives and nouns), the identification of which is necessary in order to extract the medical terms. Also, Named Entity Recognition (NER) systems may be used to detect several entities (e.g. diseases, chemicals, species or genes). Where a Named Entity Recognition system is used, this system may be a neural network trained with medical texts annotations to be able of extracting in new texts medical entities, building a Medical Named Entity Recognition (MNER) tool.

. The output from the text analyser and parser 5 is the logical components, plus a set of candidate medical terms, nouns and medical entities detected in the logical components. These outputs are then passed to the automatic coding solver 9.

. The automatic coding solver 9 receives the output from the text analyser and parser 5. A further input is received by the automatic coding solver 9 from a mapping engine 7, such as the synonym mapping engine 7 as discussed below.

. The synonym mapping engine 7 is configured to receive at least one medical classification hierarchy, as shown in step S104 of Fig. 2A, from an external source such as a hierarchy database. The medical classification hierarchy details the links between the medical standard codes in a given medical classification scheme. As discussed above, the system may be configured to use any suitable medical classification, such as the ICD scheme, and may use a plurality of medical classifications. Once retrieved, one or more medical classification hierarchy may be retained by the synonym mapping engine 7.

. Typically, the classification hierarchy is provided in the form of a knowledge graph; this is the most efficient way to detail the ontology of a medical classification hierarchy. A knowledge graph may represent the relationships between the different medical standard codes (relating to medical entities) forming a medical classification hierarchy, and may also be used to group the codes into categories, sub categories, and so on. For example, a category of "respiratory diseases" may have subcategories of "asthma", "bronchitis", and so on. In turn, the sub-category of "asthma" may have further subsidiary categories of "exercise induced asthma", "nonallergic asthma", and so on. The terms "category", "sub category", etc. are used in a nonlimiting sense, and do not imply the presence or absence of higher or subsidiary groupings.

. Each of the medical entities may be treated as a node within the knowledge graph, with the relationships between entities indicated by vertices between nodes. Knowledge graphs may be used to represent ontologies using multi-dimensional plots, that is, graphically. While the synonym mapping engine 7 may receive the knowledge graph in a graphical format, the knowledge graph may also be received in another form, for example, as tabulated information.

. When the synonym mapping engine 7 has received a medical classification hierarchy, this component is then configured to semantically annotate the knowledge graph (see step S105 of Fig. 2A). The semantic annotation comprises enhancing the medical classification hierarchy with relevant additional information, such as synonyms of terms used in the description of the medical condition to which a given code relates, alternative names for the condition (if applicable), translations of the description into other languages, and so on. The new information is linked to each medical entity in order to extend the semantic data of the knowledge graph, thereby generating an extended knowledge graph for the given medical coding hierarchy. The synonyms may be provided with reference to any suitable database, such as the Ontology of Consumer Health Vocabulary , which is a SKOS-encoded implementation of the "Open Access, Collaborative Consumer Health Vocabulary Initiative" by the University of Utah. Enhancing the knowledge graph with synonyms increases the likelihood of correct matching between medical terms detected by the text analyser and parser 5 and the medical codes in the hierarchy. The enhanced knowledge graph is passed from the synonym mapping engine 7 to the automatic coding solver 9.

. An example of a (truncated) entry in an extended knowledge graph for the condition "generalized anxiety disorder" is shown below:

> Node (label: ICD9, ICD10, etc.)
> code (e.g. F41.1)
> description_en (e.g. Generalized anxiety disorder)
> description_es (e.g. Trastorno de ansiedad generalizada)
> description_ja (e.g. 全般性不安障害)
> ...

synonyms_en (e.g. anxiety disorder generalize, anxiety disorder generalized, anxiety disorders generalized, gad, gads, generalised anxiety disorder, generalized anxiety disorders)

. The automatic coding solver 9 receives the medical terms in the plurality of logical components from the text analyser and parser 5 and the enhanced knowledge graph from the synonym mapping engine 7. The medical terms are then compared against the enhanced knowledge graph, and a list of potential matching medical standard codes is generated for each of the medical terms. The lists of potential matching medical standard codes are then compared, and top matching medical standard codes are output based on the comparison.

. Typically, the automatic coding solver 9 uses a probabilistic weighted-valued algorithm to return an ordered list of potential entity pairs of medical entity description with its standard codes (medical entity-codes); and a list of potential codes matching an input text in unstructured format (clinical note-codes). In order to improve the quality of the results generated, and increase the speed of subsequent calculations, the automatic coding solver 9 may pre-process the medical terms. This pre-processing may comprise converting the medical terms to their root terms, for example, using singular forms, lemmatization, stemming, and so on. The pre-processing may also or alternatively comprise identifying black-list terms, which may be terms that are too common in medical terminology to provide information useful for identifying potential matching medical standard codes, and removing these black-list terms before analysis.

. Once pre-processing of the medical terms has been performed (if the automatic coding solver 9 is configured to use pre-processing), the medical terms are compared against the enhanced knowledge graph for the medical standard codes of the medical hierarchy, such that potentially matching medical standard codes may be identified (see steps S106 and S107 of Fig. 2B). The comparison may utilise string matching algorithms to identify the potentially matching medical standard codes such as, for example, Jaro-Winkler distance equations for the comparison of strings. The Jaro-Winkler distance equation for comparison of the similarity of two strings $s_1$ and $s_2$ is based on the Jaro similarity of the strings, $(sim_j)$, which is given by the equation:

$$sim_j = \frac{1}{3}\left(\frac{m}{|s_1|} + \frac{m}{|s_2|} + \frac{m-t}{m}\right)$$

where m is the number of matching characters, that is, characters in $s_1$ and $s_2$ that are the same, and in the same location within the strings (a positive integer; if the number of matching characters is 0, then the value of $sim_j$ is also 0). The value of |s| is the length of string s (that is, the number of characters in the string). Two characters are considered to be matching but transposed (that is, instances where the same character appears in the two strings but in different locations) if the equation

$$\left\lfloor \frac{\max(|s_1|,|s_2|)}{2} \right\rfloor - 1 \text{ is satisfied, where } \max(|s_1|,|s_2|)$$

returns a value of the length of the longer of the two strings. The value of t is half the number of transpositions.

. The Jaro similarity is modified to obtain the Jaro-Winkler similarity $(sim_w)$. The Jaro-Winkler similarity is given by the equation:

$$sim_w = sim_j + (lp(1 - sim_j))$$

where l is the number of matching characters at the start of the two strings, to a maximum of 4 characters, and p is a scaling factor. The value of p is typically set at 0.1, and not higher than 0.25. The Jaro-Winkler similarity modifies the Jaro similarity to give greater weight to similarities at the start of pairs of strings, and is therefore helpful for identifying strings for related medical terms (for example, "bronchitis" and "bronchi"). Other string similarity measuring equations may be used, for example, Levenshtein distance measurements or Hamming distance measurements.

. In some aspects of embodiments, the results from the comparisons between the medical terms and the medical standard codes may be further tested using a predetermined threshold. That is, the similarity values calculated using (for example) the Jaro-Winkler similarity measure may be compared to a matching threshold value, with only medical standard codes that generate similarity values that match or exceed the threshold considered to be potentially matching medical standard codes. In this way, the chances of incorrect matches and corresponding potential incorrect diagnosis advice are reduced.

. In situations where similarity comparisons between a given medical term and the medical standard codes in a medical classification scheme do not result in any similarity values that satisfy the predetermined threshold, the automatic coding solver 9 may be configured to output a null result. That is, the automatic coding solver 9 may not output any potentially matching medical standard codes for the given medical term, and the given medical term may then be disregarded in subsequent processing. This configuration of the automatic coding solver 9 reduces the chances of incorrect top matching medical standard codes being output by the automatic coding solver 9 to the enrichment engine 11, but also means that information from the unstructured input may be lost (due to the medical term being essentially discarded from subsequent analysis). As an alternative response to situations where similarity comparisons between a given medical term and the medical standard codes in a medical classification scheme do not result in any similarity values that satisfy the predetermined threshold, the automatic coding solver 9 may output as a potentially matching medical standard code the medical standard code that generated the highest similarity value, despite this similarity value being below the predetermined threshold. Configuring the automatic coding solver 9 in this way reduces the chances of information from the unstructured input being lost, but may also increase the chances of an incorrect top matching medical standard code being passed to the enrichment engine 11.

. The automatic coding solver 9 may be further configured, when performing the comparison between the medical terms and the medical standard codes, to separate the potential matching medical standard codes into a number of phrase categories based on relevance. That is, once identified as a potential matching medical standard code, each of the potential matching medical standard codes may be categorised, and this category information may subsequently be used when generating the top matching medical standard codes for the medical terms. In this way, information which is likely to be of more use for generating diagnosis information is passed to the enrichment engine 11.

. Any number of categories may be used depending on the specific requirements of a given system, however large numbers of categories may result in the process of determining the top matching medical standard codes becoming excessively complex. A useful number of categories for most hierarchies is three. In an aspect of an embodiment, three categories are used: first phrases which are used in the description of a medical standard code; second phrases which are synonyms for phrases used in the description of a medical standard code; and third phrases which are general healthcare related phrases but are neither first phrases nor second phrases. In an example application of a three category system, if the description of a medical standard code for a given condition includes the phrase "abdominal pain", then potential matching medical standard code "abdominal pain" would be a first category phrase as this phrase appears directly in the description for the given condition. The potential matching medical standard code "stomach ache" is a synonym of the phrase that appears in the description, and therefore would be a second category phrase. The potential matching medical standard code "sickness" is a general phrase used in healthcare, but does not satisfy the criteria to be either a first or second category phrase for the given condition, and would therefore be a third category phrase.

. It is possible that a potential matching medical standard code may relate to several different conditions and may be a different category phrase in relation to the different conditions. In this situation, the potential matching medical standard code may be considered separately with reference to each condition when the potential matching medical standard codes are evaluated to determine top matching medical standard codes.

. Once the potential matching medical standard codes have been identified using similarity comparison and, if applicable, categorised into different phrase categories, the potential matching medical standard codes for each medical term detected in the unstructured input are then compared, as shown in step S108 of Fig. 2B. The comparison is used to produce a list of top marching medical standard codes, which is then output, as shown in step S109 of Fig. 2B. A single consolidated list of top matching codes may be generated for the unstructured input as a whole, which increases the amount of medical terms that the top matching codes are based on (because codes from the entire unstructured input are used), and therefore increases the likelihood of the top matching codes accurately reflecting the content of the unstructured input.

. A separate list of top matching codes may alternatively be generated for each logical component in the unstructured input, and the lists of top matching components may then be processed separately. Generating separate lists for the logical components increases the chance, in the event that the unstructured input relates to more than one medical condition, the different medical conditions are all detected. For example, if the unstructured input is written notes of a consultation between a medical practitioner and a patient which involved a discussion of two separate illnesses the patient is suffering from, generating separate lists of top matching components for each of the logical components (sentences in this case) of the unstructured input increases the chances of both illnesses being detected, because it is likely that at least some of the logical components will relate exclusively to only one of the illnesses. However, as the total number of detected medical terms used to generate each individual list of top matching codes is very likely to be lower than if a single consolidated list of top matching codes is generated from the unstructured input, the top matching generated codes are each based on less information and may not fully reflect the overall content of the unstructured input.

. The top matching codes are generated by consolidating the potential matching standard codes (either for an entire unstructured input or per logical component, as discussed above). The consolidation generally comprises outputting the most commonly occurring potential matching standard codes, however the weighting of the potential matching medical standard codes (as discussed above) may also be taken into consideration. Where a number of the potential matching medical standard codes relate to the same medical condition (for example, all appear in the description of that condition), these codes may also be given extra weight when determining the top matching codes. Following the determination of the top matching codes, these codes are then output to the enrichment engine 11 for further processing (see step S109 in Fig. 2B).

. An example of the process from the receiving of an unstructured input to the outputting of the top matching codes to the enrichment engine 11 is discussed below. In this example, the unstructured input is in the form of a clinical note that has been typed by a medical practitioner, in relation to the visit of a patient to a hospital. The raw unstructured input is shown below:

*This 5-year-old male presents to Childrens Hospital Emergency Department by the mother with have asthma. Mother states he has been wheezing and coughing. They saw their primary medical doctor. He was evaluated at the clinic, given the breathing treatment and discharged home, was not having asthma, prescribed prednisone and an antibiotic. They told to go to the ER if he got worse. He has had some vomiting and some abdominal pain. His peak flows on the morning are normal at 150, but in the morning, they were down to 100 and subsequently decreased to 75 over the course of the day. The differential entertained on this patient includes reactive airways disease, viral syndrome, and foreign body pneumonia. He is evaluated in the emergency department with continuous high-dose albuterol, Decadron by mouth, pulse oximetry, and close observation. Chest x-ray reveals bronchial thickening, otherwise no definite infiltrate. She is further treated in the emergency department with continued breathing treatments. At 0048 hours, he has continued tight wheezes with saturations 99%, but ED sats are 92% with coughing spells. Based on the above, the hospitalist was consulted and accepts this patient for admission to the hospital with the working diagnosis of respiratory distress and asthma.*

. As the unstructured input is already in the form of a text file, it is not necessary for the receiver 3 to perform any conversion of the unstructured input. Instead, the receiver 3 transfers the unstructured input to the text analyser and parser 5. The text analyser and parser 5 then splits the unstructured inputs into logical components, in this example, sentences. The sentences are then analysed to detect and extract medical terms and entities. A list of the extracted medical terms and entities detected by the text analyser and parser 5 in the present example is shown below (for simplicity the list is shown in the order the medical terms and entities appear in the unstructured input):

*asthma, wheezing, coughing, breathing treatment, asthma, prednisone, antibiotic, vomiting, abdominal pain, reactive airways disease, viral syndrome, foreign body pneumonia, continuous high-dose albuterol, Decadron by mouth, pulse oximetry, chest x-ray, bronchial thickening, continued breathing treatments, continued tight wheezes, coughing spells, respiratory distress, asthma.*

. The detected medical terms are then passed to the automatic coding solver 9. The automatic coding solver 9 has received a medical classification hierarchy from the synonym mapping engine, wherein the medical classification hierarchy has been enhanced as discussed above. In this example, the ICD-10 medical classification hierarchy is used.

. Having received the extracted medical terms and entities from the text analyser and parser 5, the automatic coding solver 9 in the present example pre-processes the extracted medical terms and entities. For example, the terms "wheezing" and "wheezes" would both be pre-processed to return the root term "wheeze". The automatic coding solver 9 in the present example then performs similarity comparisons for the extracted medical terms using the Jaro-Winkler similarity equation. Potential matching medical standard codes are identified based on the similarity comparisons; example results for the medical terms "asthma", "abdominal pain" and "respiratory distress" are shown below, along with the calculated $sim_w$ values:

*asthma*

- *J45 (Asthma): value 1.0*
- *J46(Status asthmaticus): value 1.0*
- *J45.0(Predominantly allergic asthma): value 0.6*

*abdominal pain*

- *R10.4(Other and unspecified abdominal pain): value 0.6*
- *R10(Abdominal and pelvic pain): value 0.4*

*respiratory distress*

- *J80(Adult respiratory distress syndrome): value 0.6*
- *P22(Respiratory distress of newborn): value 0.6*
- *P22.0(Respiratory distress syndrome of newborn): value 0.6*

. For brevity, the potential matching medical standard codes for all of the medical terms are not shown. In the examples shown above, both categories (such as J45 and R10) and corresponding sub categories (such as J45.0 and R10.4 respectively) are present; the automatic coding solver 9 may link or consolidate these terms when determining and outputting the top matching medical standard codes. Also, the results shown above have already been subjected to the predetermined threshold as discussed above; results which did not satisfy the threshold are not presented.

. In the present example, the automatic coding solver 9 is configured to output top matching medical standard codes for the unstructured input as a whole. Based on an analysis of all of the potential matching medical standard codes, the automatic coding solver 9 outputs the following top matching medical standard codes for the unstructured input:

- *J45(Asthma)*
- *J80(Adult respiratory distress syndrome)*
- *R10.4(Other and unspecified abdominal pain)*

. The above example considers a situation in which three top matching medical standard codes are output by the automatic coding solver 9; larger or smaller numbers of codes may be output depending on the specific system configuration.

. The output from the automatic coding solver 9 is passed to the enrichment engine 11 (as discussed above, see step S109 in Fig. 2B). The enrichment engine 11 comprises or is connected to a database 13 linking diagnoses, symptoms and treatments. Typically, the diagnoses in the database 13 are linked to corresponding medical standard codes using the same medical hierarchy as used by the synonym mapping engine 7 and automatic coding solver 9. In examples wherein the database 13 used by the enrichment engine 11 comprises medical standard codes from a different medical hierarchy to those used by the synonym mapping engine 7 and automatic coding solver 9, or the database 13 does not comprise medical standard codes, it is necessary to retrieve information from the database 13 using medical condition names or a conversion table converting between different medical hierarchies; both of these options are less efficient than using the same medical hierarchy in the database 13, the synonym mapping engine 7 and the automatic coding solver 9.

. The database 13 may be compiled from collected and anonymised patient health records, medical textbooks and journal papers, entries by medical personnel, and so on. Each entry for a given medical condition (such as a disease) may be enhanced with information on related conditions, such as: conditions which may be mistaken in medical examination for the given medical condition; conditions which may trigger development of the given medical condition; conditions which may result from the given medical condition, and so on. The entries may also be linked to information on symptoms which may be used to help diagnose given medical conditions, and treatments which a medical practitioner may apply in order to relieve the symptoms of a given medical condition and/or cure the medical condition. Further information may also be provided if available, for example, if various treatments are available and are suitable for different types of patients (such as patients having certain allergies, infants, expectant mothers, and so on), this information may also be provided. The enrichment engine 11 may be configured to compile the database 13 from individually entered pieces of information (such as anonymised patient health records), or a completed database may be uploaded to the enrichment engine 11.

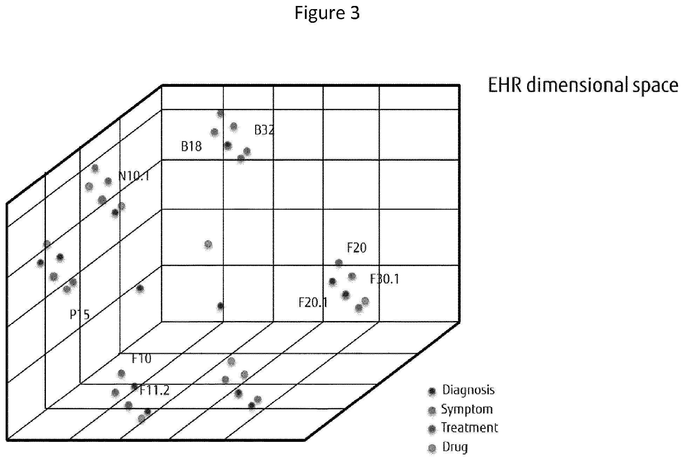

. The database 13 may be stored in any suitable format. One option is to use a series of anonymised patient health records to form a word embedding matrix, containing diagnoses, treatments, symptoms, procedures, drugs, etc. from the anonymised patient health records, and then using the word embedding matrix to create a biomedical entity matrix that links together the diagnoses, treatments, symptoms, procedures, drugs, etc. in a format that may be searched using the top matching medical standard codes. An example of a biomedical entity matrix is shown in Fig. 3. A biomedical entity matrix may be created using, for example, a skip-gram model. The skip-gram is model architecture for word embedding (i.e. to define distributed and correlated representation of words). The main characteristic of skip-gram model when iterating over the words of each sentence is that it uses the current word to predict its neighbours and in this way try to know the context.

. When the enrichment engine 11 receives the top matching medical standard codes relating to a given unstructured input, the enrichment engine 11 queries the database 13 using the top matching medical standard codes and retrieves diagnoses, symptom and treatment information relating to the top matching medical standard codes (see step S110 in Fig. 2B). The enrichment engine 11 may then simply output this information for consideration by medical personnel, as shown in step S111 of Fig. 2B. The information may be useful in suggesting further diagnostic steps that may be taken to confirm a diagnoses, suggesting treatment options, and so on. In this way, a diagnostic aid 1 is provided which may both guide the deliberations of medical personnel and provide additional information rapidly and minimising the need for laborious research.

. In addition to simply outputting retrieved from the database 13 based on information based on the top matching medical standard codes (as discussed above), the enrichment engine 11 may also be linked to other information sources, such as a patient records repository. In some aspects of embodiments, the enrichment engine 11 may access the specific medical record linked to a patient that is the subject of the unstructured input, and to output the specific medical record in conjunction with the output diagnoses, symptoms and treatments. The provision of patient specific data may help guide medical personnel in treating a condition by, for example, indicating if a patient has a history of similar symptoms.

. Where the enrichment engine 11 has access to a patient records repository, the enrichment engine 11 may be configured to analyse a specific medical record for a patient, to extract medical standard codes from the specific medical record, and to take the extracted medical standard codes into account when outputting the diagnoses, symptoms and treatments. The enrichment engine 11 may be further or alternatively configured to update the specific medical record with the output diagnoses, symptoms and treatments. By providing information based on the specific patient medical record, and/or by updating the record, the utility of the diagnostic aid 1 is further increase and the time of medical personnel is saved.

. In order to allow medical personnel to more easily verify the information provided by the enrichment engine 11, and to allow medical personnel to determine if further factors should be considered, the enrichment engine 11 may be further configured to output the unstructured input in conjunction with the diagnoses, symptoms and treatments for consideration and evaluation by medical personnel. In this way, medical personnel may detect potentially pertinent information which may have been overlooked or misinterpreted by the diagnostic aid 1.

. The diagnostic aid 1 may be further configured to store the unstructured input in conjunction with the output diagnoses, symptoms and treatments. The storage 15 may form part of the diagnostic aid 1, or may be a separate storage unit connected to the diagnostic aid 1 by a suitable data connection, such as the internet. In the aspect of an embodiment shown in Fig. 1, the storage 15 forms part of the diagnostic aid 1. The combined information may be useful for performing reviews of historic performance of the system, or alternatively may form a training data set for use in training a further system.

. The diagnostic aid 1 and method for assisting medical personnel in performing a diagnosis may automate the analysis of unstructured inputs, and may therefore save time and effort for the medical practitioner, and also provide the medical practitioner with information which may not otherwise have been discovered by the medical practitioner.

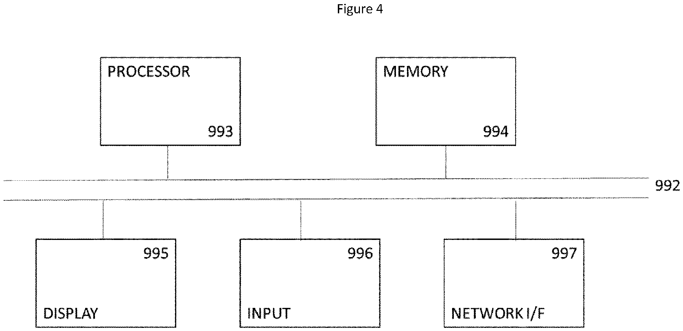

. Fig. 4 is a block diagram of a computing device, such as a personal computer, which embodies an example, and which may be used to implement an embodiment of the method for assisting medical personnel in performing a diagnosis. The computing device comprises a processor 993, and memory 994. Optionally, the computing device also includes a network interface 997 for communication with other computing devices, or for communicating with remote databases.

. An example may be composed of a network of such computing devices, such that components of the diagnostic aid 1 are split across a plurality of computing devices. Optionally, the computing device also includes one or more input mechanisms such as keyboard and mouse or touchscreen interface 996, and a display unit such as one or more monitors 995. The components are connectable to one another via a bus 992.

. The memory 994 may include a computer readable medium, which term may refer to a single medium or multiple media (e.g., a centralized or distributed database and/or associated caches and servers) configured to carry computer-executable instructions or have data structures stored thereon. The memory 994 may be the same memory 9 as may be used for the storage, or a separate memory. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices). In particular, the computer readable medium may comprise a computer program which, when executed on a computer, causes the computer to perform a method for assisting medical personnel in performing a diagnosis as discussed above.

. The processor 993 is configured to control the computing device and execute processing operations, for example executing code stored in the memory to implement the various different functions of the receiver 3, text analyser and parser 5, synonym mapping engine 7, automatic coding solver 9 and enrichment engine 11 described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

. The display unit 997 may display a representation of data stored by the computing device and may also display a cursor and dialog boxes and screens enabling interaction between a user and the programs and data stored on the computing device. The display unit may also comprise a touchscreen interface. The input mechanisms 996 may enable a user to input data and instructions to the computing device.

. The network interface (network I/F) 997 may be connected to a network, such as the Internet, and is connectable to other such computing devices via the network. The network I/F 997 may control data input/output from/to other apparatus via the network. The network interface may also be used in receiving unstructured inputs, receiving medical classification hierarchies, transmitting diagnoses, symptoms and treatments, and so on.

. Other peripheral devices such as microphone, speakers, printer, power supply unit, fan, case, scanner, trackerball etc. may be included in the computing device.

. The receiver 3 of Figure 1 may be a processor 993 (or plurality thereof) executing processing instructions (a program) stored on a memory 994 and exchanging data via a network I/F 997 or bus 992. In particular, the processor 993 may execute processing instructions to receive an unstructured input. Furthermore, the processor 993 may execute processing instructions to send the unstructured input to other components within the diagnostic aid 1, such as the text analyser and parser 5, or to store the unstructured input in the storage.

. The text analyser and parser 5 of Figure 1 may be a processor 993 (or plurality thereof) executing processing instructions (a program) stored on a memory 994 and exchanging data via a network I/F 997 or bus 992. In particular, the processor 993 may execute processing instructions to split the unstructured input into a plurality of logical components, and to detect medical terms in the plurality of logical components as discussed above. Furthermore, the processor 993 may execute processing instructions to send an output to other components within the diagnostic aid 1, such as the automatic coding solver 9.

. The synonym mapping engine 7 of Figure 1 may be a processor 993 (or plurality thereof) executing processing instructions (a program) stored on a memory 994 and exchanging data via a network I/F 997 or bus 992. In particular, the processor 993 may execute processing instructions to receive a medical classification hierarchy of medical standard codes in the form of a knowledge graph, and semantically annotate the knowledge graph with synonyms of medical terms used in the medical standard codes as discussed above. Furthermore, the processor 993 may execute processing instructions to send the knowledge graph to other components within the diagnostic aid 1, such as the automatic coding solver 9.

. The automatic coding solver 9 of Figure 1 may be a processor 993 (or plurality thereof) executing processing instructions (a program) stored on a memory 994 and exchanging data via a network I/F 997 or bus 992. In particular, the processor 993 may execute processing instructions analyse the medical terms detected in the plurality of logical components, to generate a list of potential matching medical standard codes for each of the medical terms, to compare the lists of potential matching medical standard codes, and to output top matching medical standard codes based on the comparison as discussed above. Furthermore, the processor 993 may execute processing instructions to send the top matching medical standard codes to other components within the apparatus 1, such as the enrichment engine 11.

. The enrichment engine 11 of Figure 1 may be a processor 993 (or plurality thereof) executing processing instructions (a program) stored on a memory 994 and exchanging data via a network I/F 997 or bus 992. In particular, the processor 993 may execute processing instructions to compare the top matching medical standard codes output by the automatic coding solver 9 against entries in the database of diagnoses. Furthermore, the processor 993 may execute processing instructions to output diagnoses, symptoms and treatments linked to each of the top matching medical standards codes for assisting medical personnel in providing a diagnosis, and/or to store the output diagnoses, symptoms and treatments in a storage 15.

. Exemplary methods may be carried out on one or more computing devices such as that illustrated in Figure 4. Such a computing device need not have every component illustrated in Figure 4, and may be composed of a subset of those components. A method may be carried out by a single computing device in communication with one or more data storage servers via a network, as discussed above. The scope of the invention is defined by the claims.

**Claims**

1. A medical diagnostic apparatus (1) for assisting medical personnel in performing a diagnosis, the diagnostic apparatus (1) comprising:

   a receiver (3) configured to receive an unstructured input;
   an analyser and parser (5) configured to receive the unstructured input from the receiver (3), to split the unstructured input into a plurality of logical components, wherein the logical components are term groupings, and to detect medical terms in the plurality of logical components using a neural network;
   a mapping engine (7) configured to receive a medical classification hierarchy of medical standard codes in the form of a knowledge graph, and semantically annotate the knowledge graph with synonyms of medical terms used in the medical standard codes to create an enhanced knowledge graph;

an automatic coding solver (9) configured: to receive the enhanced knowledge graph from the mapping engine (7); to compare the medical terms detected in the plurality of logical components by the analyser and parser (5) against the enhanced knowledge graph using a string matching algorithm; to generate a list of potential matching medical standard codes for each of the medical terms based on the comparison with the enhanced knowledge graph; to compare the lists of potential matching medical standard codes with each other; and to output top matching medical standard codes based on the most commonly occurring potential matching medical standard codes identified in the comparison of the potential matching medical standard codes; and

an enrichment engine (11) including a database of diagnoses linked to symptoms and treatments (13), wherein the enrichment engine (11) is configured to compare the top matching medical standard codes output by the automatic coding solver (9) against entries in the database of diagnoses (13), and to output diagnoses, symptoms and treatments related to each of the top matching medical standards codes for assisting medical personnel in providing a diagnosis.

2. The medical diagnostic apparatus (1) of claim 1, wherein the automatic coding solver (9) is further configured to apply a matching threshold that is a threshold for the similarity between two potential matching medical standard codes when comparing the lists of potential matching medical standard codes.

3. The medical diagnostic apparatus (1) of claim 2 wherein, if none of the comparisons of potential matching medical standard codes satisfies the threshold, a null result is output to the enrichment engine (11) as the top matching medical standard code comparison result.

4. The medical diagnostic apparatus (1) of claim 2 wherein, if none of the comparisons of potential matching medical standard codes satisfies the threshold, the highest ranking comparison below the threshold is output to the enrichment engine (11) as the top matching medical standard code comparison result.

5. The medical diagnostic apparatus (1) of any preceding claim, wherein the enrichment engine (11) is further configured to access a specific medical record linked to a patient that is the subject of the unstructured input, and to output the specific medical record in conjunction with the output diagnoses, symptoms and treatments.

6. The medical diagnostic apparatus (1) of claim 5, wherein the enrichment engine (11) is further configured to analyse the specific medical record, to extract medical standard codes from the specific medical record, and to take the extracted medical standard codes into account when outputting the diagnoses, symptoms and treatments.

7. The medical diagnostic apparatus (1) of any of claims 5 and 6, wherein the enrichment engine (11) is further configured to update the specific medical record with the output diagnoses, symptoms and treatments.

8. The medical diagnostic apparatus (1) of any preceding claim, wherein the enrichment engine (11) is further configured to output the unstructured input for evaluation by medical personnel.

9. The medical diagnostic apparatus (1) of claim 8, wherein the output diagnoses, symptoms and treatments and unstructured input are stored in a storage device (15), for use in a training data set.

10. The medical diagnostic apparatus (1) of any preceding claim, wherein

either the automatic coding solver (9) is configured to output top matching codes separately for each of the logical components based on the most commonly occurring potential matching medical standard codes identified for each of the logical components, and the enrichment engine (11) is configured to compare the top matching medical standard codes output by the automatic coding solver against entries in the database of diagnoses (13) separately for each of the logical components, and to output diagnoses, symptoms and treatments linked to each of the top matching medical standards codes separately for each of the logical components, or

the automatic coding solver (9) is configured to output consolidated top matching codes for the unstructured input as a whole based on the most commonly occurring potential matching medical standard codes identified for the unstructured input, and the enrichment engine (11) is configured to compare the top matching medical standard codes output by the automatic coding solver (9) against entries in the database of diagnoses (13), and to output diagnoses, symptoms and treatments linked to the top matching medical standards codes, for the unstructured input as a whole.

11. The medical diagnostic apparatus (1) of any preceding claim wherein the automatic coding solver (9) is configured:

when analysing the potential matching medical standard codes, to divide the potential matching medical standard codes into phrase categories: first phrases used in the description of a medical standard code; second phrases that are synonyms of terms used in the description of a medical standard code; and third phrases that are healthcare related phrases, but are neither first phrases or second phrases, and
when generating a list of top matching medical standard codes, the first phrases are given greater weight than the second phrases, and the second phrases are given greater weight than the third phrases.

12. The medical diagnostic apparatus (1) of any of claims 1 to 11, wherein the unstructured input received by the receiver (3) is an audio file containing a recording of a patient consultation, and the receiver (3) comprises a transcription module (4) configured to generate a text file based on the audio file.

13. A computer implemented
method for assisting medical personnel in performing a diagnosis, the method comprising:

receiving an unstructured input;
splitting the received unstructured input into a plurality of logical components, wherein the logical components are term groupings, and detecting medical terms in the plurality of logical components using a neural network;
receiving a medical classification hierarchy of medical standard codes in the form of a knowledge graph, and semantically annotating the knowledge graph with synonyms of medical terms used in the medical standard codes to create an enhanced knowledge graph;
comparing the medical terms detected in the plurality of logical components against the enhanced knowledge graph using a string matching algorithm, generating a list of potential matching medical standard codes for each of the medical terms based on the comparison with the enhanced knowledge graph, comparing the lists of potential matching medical standard codes with each other, and outputting top matching medical standard codes based on the most commonly occurring potential matching medical standard codes identified in the comparison of the potential matching medical standard codes; comparing the top matching medical standard codes output against entries in a database of diagnoses linked to symptoms and treatments, and outputting diagnoses, symptoms and treatments related to each of the top matching medical standards codes for assisting medical personnel in providing a diagnosis.

14. A non-transitory computer readable medium comprising code which, when executed by a computer, causes the computer to execute the method of claim 13.

**Patentansprüche**

1. Medizinische Diagnosevorrichtung (1) zum Unterstützen von medizinischem Personal beim Durchführen einer Diagnose, wobei die Diagnosevorrichtung (1) Folgendes umfasst:

einen Empfänger (3), der zum Empfangen einer unstrukturierten Eingabe konfiguriert ist;
einen Analysator und Parser (5), der zum Empfangen der unstrukturierten Eingabe von dem Empfänger (3), Aufteilen der unstrukturierten Eingabe in eine Vielzahl von logischen Komponenten, wobei die logischen Komponenten Begriffsgruppierungen sind, und Erkennen von medizinischen Begriffen in der Vielzahl von logischen Komponenten unter Verwendung eines neuronalen Netzes konfiguriert ist;
eine Zuordnungs-Engine (7), die zum Empfangen einer medizinischen Klassifikationshierarchie von medizinischen Standardcodes in Form eines Wissensgraphen und semantischen Versehen des Wissensgraphen mit Synonymen von in den medizinischen Standardcodes verwendeten medizinischen Begriffen zum Erstellen eines erweiterten Wissensgraphen konfiguriert ist;
einen automatischen Code-Löser (9), der zu Folgendem konfiguriert ist: Empfangen des erweiterten Wissensgraphen von der Zuordnungs-Engine (7); Vergleichen der medizinischen Begriffe, die in der Vielzahl von logischen Komponenten durch den Analysator und Parser (5) erkannt wurden, mit dem erweiterten Wissensgraphen unter Verwendung eines String-Matching-Algorithmus; Erzeugen einer Liste mit potenziell übereinstimmenden medizinischen Standardcodes für jeden der medizinischen Begriffe auf der Grundlage des Vergleichs mit dem erweiterten Wissensgraphen; Vergleichen der Listen mit potenziell übereinstimmenden medizinischen Standardcodes miteinander; und Ausgeben der am besten übereinstimmenden medizinischen Standardcodes auf der Grundlage der am häufigsten auftretenden potenziell übereinstimmenden medizinischen Standardcodes, die bei dem Vergleich der potenziell übereinstimmenden medizinischen Standardcodes identifiziert wurden; und eine Anreicherungs-Engine (11), die eine Datenbank von mit Symptomen und Behandlungen verknüpften Diagnosen (13) beinhaltet, wobei die Anreicherungs-Engine (11) zum Vergleichen der von dem automatischen Code-Löser (9) ausgegebenen am besten übereinstimmenden medizinischen Standardcodes mit Einträgen in der Datenbank von Diagnosen (13) und Ausgeben von Diagnosen, Symptomen und Behandlungen, die sich auf jeden der am besten übereinstimmenden medizinischen Standardcodes beziehen, zum Unterstützen von medizinischem Personal beim Bereitstellen einer Diagnose konfiguriert ist.

2. Medizinische Diagnosevorrichtung (1) nach Anspruch 1, wobei der automatische Code-Löser (9) ferner zum Anwenden eines Übereinstimmungsschwellenwerts, der ein Schwellenwert für die Ähnlichkeit zwischen zwei potenziell übereinstimmenden medizinischen Standardcodes ist, beim Vergleichen der Listen der potenziell übereinstimmenden medizinischen Standardcodes konfiguriert ist.

3. Medizinische Diagnosevorrichtung (1) nach Anspruch 2, wobei als am besten übereinstimmender medizinischer Standardcode ein Nullergebnis als Ergebnis des Vergleichs an die Anreicherungs-Engine (11) ausgegeben wird, wenn keiner der Vergleiche potenziell übereinstimmender medizinischer Standardcodes den Schwellenwert erfüllt.

4. Medizinische Diagnosevorrichtung (1) nach Anspruch 2, wobei als am besten übereinstimmender medizinischer Standardcode der oberste Vergleich unmittelbar unter dem Sehwellenwert als Ergebnis des Vergleichs an die Anreicherungs-Engine (11) ausgegeben wird, wenn keiner der Vergleiche potenziell übereinstimmender medizinischer Standardcodes den Schwellenwert erfüllt.

5. Medizinische Diagnosevorrichtung (1) nach einem der vorhergehenden Ansprüche, wobei die Anreicherung-Engine (11) ferner zum Zugreifen auf eine spezifische Krankenakte, die mit einem Patienten verknüpft ist, der Gegenstand der unstrukturierten Eingabe ist, und Ausgeben der spezifischen Krankenakte in Verbindung mit den ausgegebenen Diagnosen, Symptomen und Behandlungen konfiguriert ist.

6. Medizinische Diagnosevorrichtung (1) nach Anspruch 5, wobei die Anreicherungs-Engine (11) ferner zum Analysieren der spezifischen Krankenakte, Extrahieren von medizinischen Standardcodes aus der spezifischen Krankenakte und Berücksichtigen der extrahierten medizinischen Standardcodes beim Ausgeben der Diagnosen, Symptome und Behandlungen konfiguriert ist.

7. Medizinische Diagnosevorrichtung (1) nach einem der Ansprüche 5 und 6, wobei die Anreicherungs-Engine (11) ferner zum Aktualisieren der spezifischen Krankenakte mit den ausgegebenen Diagnosen, Symptomen und Behandlungen konfiguriert ist.

8. Medizinische Diagnosevorrichtung (1) nach einem der vorhergehenden Ansprüche, wobei die Anreicherungs-Engine (11) ferner zum Ausgeben der unstrukturierten Eingabe zum Auswerten durch medizinisches Personal konfiguriert ist.

9. Medizinische Diagnosevorrichtung (1) nach Anspruch 8, wobei die ausgegebenen Diagnosen, Symptome und Behandlungen und die unstrukturierte Eingabe in einer Speichervorrichtung (15) zur Verwendung in einem Trainingsdatensatz gespeichert sind.

10. Medizinische Diagnosevorrichtung (1) nach einem der vorhergehenden Ansprüche, wobei

entweder der automatische Code-Löser (9) zum Ausgeben von am besten übereinstimmenden Codes getrennt für jede der logischen Komponenten auf der Grundlage der am häufigsten auftretenden potenziell übereinstimmenden medizinischen Standardcodes, die für jede der logischen Komponenten identifiziert wurden, konfiguriert ist und die Anreicherungs-Engine (11) zum Vergleichen der von dem automatischen Code-Löser ausgegebenen am besten übereinstimmenden medizinischen Standardcodes getrennt für jede der logischen Komponenten mit Einträgen in der Datenbank von Diagnosen (13) und Ausgeben von Diagnosen, Symptomen und Behandlungen, die mit jedem der am besten übereinstimmenden medizinischen Standardcodes verknüpft sind, getrennt für jede der logischen Komponenten konfiguriert ist oder der automatische Code-Löser (9) zum Ausgeben von konsolidierten am besten übereinstimmenden Codes für die unstrukturierte Eingabe als Ganzes auf der Grundlage der am häufigsten auftretenden potenziell übereinstimmenden medizinischen Standardcodes, die für die unstrukturierte Eingabe identifiziert wurden, konfiguriert ist und die Anreicherungs-Engine (11) zum Vergleichen der von dem automatischen Code-Löser (9) ausgegebenen am besten übereinstimmenden medizinischen Standardcodes mit Einträgen in der Datenbank von Diagnosen (13) und Ausgeben von Diagnosen, Symptomen und Behandlungen, die mit den am besten übereinstimmenden medizinischen Standardcodes verknüpft sind, für die unstrukturierte Eingabe als Ganzes konfiguriert ist.

11. Medizinische Diagnosevorrichtung (1) nach einem der vorhergehenden Ansprüche, wobei der automatische Code-Löser (9) zu Folgendem konfiguriert ist:

Unterteilen der potenziell übereinstimmenden medizinischen Standardcodes in Phrasenkategorien beim Analysieren der potenziell übereinstimmenden medizinischen Standardcodes: erste Phrasen, die in der Beschreibung eines medizinischen Standardcodes verwendet werden; zweite Phrasen, die Synonyme von Begriffen sind, die in der Beschreibung eines medizinischen Standardcodes verwendet werden; und dritte Phrasen, die mit der medizinischen Versorgung zusammenhängen, aber weder erste noch zweite Phrasen sind, und stärkeres Gewichten die ersten Phrasen als die zweiten Phrasen und stärkeres Gewichten der zweiten Phrasen als der dritten Phrasen beim Erstellen einer Liste von am besten übereinstimmenden medizinischen Standardcodes.

12. Medizinische Diagnosevorrichtung (1) nach einem der Ansprüche 1 bis 11, wobei die von dem Empfänger (3) empfangene unstrukturierte Eingabe eine Audiodatei ist, die eine Aufzeichnung einer Patientenkonsultation enthält, und der Empfänger (3) ein Transkriptionsmodul (4) umfasst, das zum Erzeugen einer Textdatei auf der Grundlage der Audiodatei konfiguriert ist.

13. Computerimplementiertes Verfahren zum Unterstützen von medizinischem Personal beim Durchführen einer Diagnose, wobei das Verfahren Folgendes umfasst:

Empfangen einer unstrukturierten Eingabe; Aufteilen der empfangenen unstrukturierten Eingabe in eine Vielzahl von logischen Komponenten, wobei die logischen Komponenten Begriffsgruppierungen sind, und Erkennen von medizinischen Begriffen in der Vielzahl von logischen Komponenten unter Verwendung eines neuronalen Netzes; Empfangen einer medizinischen Klassifikationshierarchie von medizinischen Standardcodes in Form eines Wissensgraphen und semantisches Versehen des Wissensgraphen mit Synonymen von in den medizinischen Standardcodes verwendeten medizinischen Begriffen zum Erstellen eines erweiterten Wissensgraphen; Vergleichen der medizinischen Begriffe, die in der Vielzahl von erkannten logischen Komponenten mit dem erweiterten Wissensgraphen unter Verwendung eines String-Matching-Algorithmus, Erzeugen einer Liste mit potenziell übereinstimmenden medizinischen Standardcodes für jeden der medizinischen Begriffe auf der Grundlage des Vergleichs mit dem erweiterten Wissensgraphen, Vergleichen der Listen mit potenziell übereinstimmenden medizinischen Standardcodes miteinander und Ausgeben der am besten übereinstimmenden medizinischen Standardcodes auf der Grundlage der am häufigsten auftretenden potenziell übereinstimmenden medizinischen Standardcodes, die bei dem Vergleich der potenziell übereinstimmenden medizinischen Standardcodes identifiziert wurden; Vergleichen der ausgegebenen am besten übereinstimmenden medizinischen Standardcodes mit Einträgen in einer Datenbank von mit Symptomen und Behandlungen verknüpften Diagnosen und Ausgeben von Diagnosen, Symptomen und Behandlungen, die sich auf jeden der am besten übereinstimmenden medizinischen Standardcodes beziehen, zum Unterstützen von medizinischem Personal beim Bereitstellen einer Diagnose.

14. Nichttransitorisches computerlesbares Medium, umfassend Code, der beim Ausführen durch einen Computer den Computer zum Ausführen des Verfahrens nach Anspruch 13 veranlasst.

**Revendications**

1. Un appareil de diagnostic médical (1) destiné à aider le personnel médical à réaliser un diagnostic, l'appareil de diagnostic (1) comprenant :

un récepteur (3) configuré pour recevoir une entrée non structurée ; un analyseur et un parseur (5) configurés pour recevoir l'entrée non structurée du récepteur (3), pour diviser l'entrée non structurée en une pluralité de composants logiques, dans lequel les composants logiques sont des groupements de termes, et pour détecter des termes médicaux dans la pluralité de composants logiques à l'aide d'un réseau neuronal ; un moteur de cartographie (7) configuré pour recevoir une hiérarchie de classification médicale de codes médicaux standard sous la forme d'un graphe de connaissances, et annoter sémantiquement le graphe de connaissances avec des synonymes de termes médicaux utilisés dans les codes médicaux standard pour créer un graphe de connaissances amélioré ;

un solveur de codage automatique (9) configuré : pour recevoir le graphe de connaissances amélioré du moteur de cartographie (7) ; pour comparer les termes médicaux détectés dans la pluralité de composants logiques par l'analyseur et le parseur (5) au graphe de connaissances amélioré à l'aide d'un algorithme de correspondance de chaînes ; pour générer, pour chacun des termes médicaux, une liste de codes médicaux standard de correspondance potentiels sur la base de cette comparaison ;

pour comparer les listes ainsi obtenues entre elles ; et pour générer les codes médicaux standard de correspondance les plus élevés sur la base des codes potentiels les plus fréquemment identifiés lors de la comparaison ; et

un moteur d'enrichissement (11) comportant une base de données de diagnostics liés à des symptômes et des traitements (13), dans lequel le moteur d'enrichissement (11) étant configuré pour comparer les codes médicaux standard de correspondance les plus élevés produits par le solveur de codage automatique (9) aux entrées de la base de données de diagnostics (13), et pour produire des diagnostics, des symptômes et des traitements liés à chacun des codes médicaux standard de correspondance les plus élevés pour aider le personnel médical à fournir un diagnostic.

2. L'appareil de diagnostic médical (1) selon la revendication 1, dans lequel le solveur de codage automatique (9) est également configuré pour appliquer un seuil de correspondance qui est un seuil pour la similarité entre deux codes médicaux standard potentiellement correspondants lors de la comparaison des listes de codes médicaux standard potentiellement correspondants.

3. L'appareil de diagnostic médical (1) selon la revendication 2, dans lequel, si aucune des comparaisons de codes médicaux standard correspondants potentiels ne satisfait le seuil, un résultat nul est envoyé au moteur d'enrichissement (11) en tant que résultat de comparaison des codes médicaux standard de correspondance les plus élevés.

4. L'appareil de diagnostic médical (1) selon la revendication 2, dans lequel, si aucune des comparaisons de codes médicaux standard correspondants potentiels ne satisfait le seuil, la comparaison la plus élevée en dessous du seuil est envoyée au moteur d'enrichissement (11) en tant que résultat de comparaison des codes médicaux standard de correspondance les plus élevés.

5. L'appareil de diagnostic médical (1) selon une quelconque revendication précédente, dans lequel le moteur d'enrichissement (11) est également configuré pour accéder à un dossier médical spécifique lié à un patient qui est le sujet de l'entrée non structurée, et pour sortir le dossier médical spécifique en conjonction avec les diagnostics, symptômes et traitements de sortie.

6. L'appareil de diagnostic médical (1) selon la revendication 5, dans lequel le moteur d'enrichissement (11) est également configuré pour analyser le dossier médical spécifique, pour extraire des codes médicaux standard du dossier médical spécifique et pour prendre en compte les codes médicaux standard extraits lors de la sortie des diagnostics, des symptômes et des traitements.

7. L'appareil de diagnostic médical (1) selon l'une quelconque des revendications 5 et 6, dans lequel le moteur d'enrichissement (11) est également configuré pour mettre à jour le dossier médical spécifique avec les diagnostics, symptômes et traitements de sortie.

8. L'appareil de diagnostic médical (1) selon une quelconque revendication précédente, dans lequel le moteur d'enrichissement (11) est également configuré pour générer l'entrée non structurée à des fins d'évaluation par le personnel médical.

9. L'appareil de diagnostic médical (1) selon la revendication 8, dans lequel les diagnostics de sortie, les symptômes et les traitements et les entrées non structurées sont stockés dans un dispositif de stockage (15), pour être utilisés dans un ensemble de données d'apprentissage.

10. L'appareil de diagnostic médical (1) selon une quelconque revendication précédente, dans lequel

soit le solveur de codage automatique (9) est configuré pour générer les codes médicaux standard de correspondance les plus élevés séparément pour chacun des composants logiques, sur la base des codes médicaux standard de correspondance potentiels les plus courants identifiés pour chacun desdits composants logiques ; et le moteur d'enrichissement (11) est configuré pour comparer les codes médicaux standard de correspondance les plus élevés générés par le solveur de codage automatique aux entrées de la base de données de diagnostics (13), séparément pour chacun des composants logiques ; et pour générer les diagnostics, les symptômes et les traitements liés à chacun des codes médicaux standard de correspondance les plus élevés, séparément pour chacun des composants logiques ; ou

soit le solveur de codage automatique (9) est configuré pour générer les codes médicaux standard de correspondance les plus élevés consolidés pour l'ensemble de l'entrée non structurée, sur la base des codes médicaux standard de correspondance potentiels les plus courants identifiés pour ladite entrée non structurée ; et le moteur d'enrichissement (11) est configuré pour comparer les codes médicaux standard de correspondance les plus élevés générés par le solveur de codage automatique (9) aux entrées de la base de données de diagnostics (13) ; et pour générer les diagnostics, les symptômes et les traitements liés auxdits codes médicaux standard de correspondance les plus élevés, pour l'ensemble de l'entrée non structurée.

11. L'appareil de diagnostic médical (1) selon une quelconque revendication précédente, dans lequel le solveur de codage automatique (9) est configuré :

lors de l'analyse des codes médicaux standard correspondants potentiels, diviser les codes médicaux standard correspondants potentiels en catégories de phrases : les premières phrases utilisées dans la description d'un code médical standard ; les deuxièmes phrases qui sont des synonymes de termes utilisés dans la description d'un code médical standard ; et les troisièmes phrases qui sont des phrases liées aux soins de santé, mais qui ne sont ni des premières ni des deuxièmes phrases, et

lors de la génération d'une liste des codes médicaux standard de correspondance les plus élevés, les premières phrases ont plus de poids que les deuxièmes phrases, et les deuxièmes phrases ont plus de poids que les troisièmes phrases.

12. L'appareil de diagnostic médical (1) selon l'une quelconque des revendications 1 à 11, dans lequel l'entrée non structurée reçue par le récepteur (3) est un fichier audio contenant un enregistrement d'une consultation de patient, et le récepteur (3) comprend un module de transcription (4) configuré pour générer un fichier texte basé sur le fichier audio.

13. Un procédé mis en œuvre par ordinateur pour aider le personnel médical à réaliser un diagnostic, le procédé comprenant :

la réception d'une entrée non structurée ;

la division de l'entrée non structurée reçue en plusieurs composants logiques, dans lequel les composants logiques sont des regroupements de termes, et la détection des termes médicaux dans la pluralité de composants logiques à l'aide d'un réseau neuronal ;

la réception d'une hiérarchie de classification médicale de codes médicaux standard sous la forme d'un graphe de connaissances, et l'annotation sémantique du graphe de connaissances avec des synonymes de termes médicaux utilisés dans les codes médicaux standard afin de créer un graphe de connaissances amélioré ;

la comparaison des termes médicaux détectés dans la pluralité de composants logiques au graphe de connaissances amélioré à l'aide d'un algorithme de correspondance de chaînes ;

la génération, pour chacun des termes médicaux, d'une liste de codes médicaux standard de correspondance potentiels sur la base de cette comparaison au graphe de connaissances amélioré ; la comparaison des listes ainsi obtenues entre elles ; et la génération des codes médicaux standard de correspondance les plus élevés sur la base des codes potentiels les plus fréquemment identifiés lors de cette comparaison ;

la comparaison des codes médicaux standard de correspondance les plus élevés aux entrées d'une base de données de diagnostics liés à des symptômes et à des traitements ; et la génération de diagnostics, de symptômes et de traitements liés à chacun desdits codes médicaux standard de correspondance les plus élevés, afin d'aider le personnel médical à fournir un diagnostic.

14. Un support lisible par ordinateur non transitoire comprenant un code qui, lorsqu'il est exécuté par un ordinateur, amène l'ordinateur à exécuter le procédé selon la revendication 13.

Figure 1

Diagnostic Aid 1

Unstructured Input

Text Analyser and Parser 5

Transcription Module 4

Receiver 3

Synonym Mapping Engine 7

Automatic Coding Solver 9

Database 13

Enrichment Engine 11

Output

Storage 15

Medical Classification Hierarchy

Figure 2A

| S101 – Receive Unstructured Input |
| S102 – Split Unstructured Input |
| S103 – Detect Medical Terms |
| S104 – Receive Medical Classification Hierarchy |
| S105 – Semantically Annotate Knowledge Graph |

(A)

Figure 2B

(A)

S106 – Analyse Medical Terms

S107 – Generate Lists of Potential Matching Medical Standard Codes

S108 – Compare Lists

S109 – Output Top Matching Medical Standard Codes

S110 – Compare Top Matching Medical Standard Codes

S111 – Output Diagnoses, Symptoms and Treatments

Figure 3

EHR dimensional space

- Diagnosis
- Symptom
- Treatment
- Drug

Figure 4

| PROCESSOR | | MEMORY | |
| | 993 | | 994 |

992

| 995 | | 996 | | 997 | |
| | | | | | |
| DISPLAY | | INPUT | | NETWORK I/F | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150095016 A1